# EUROPEAN PATENT SPECIFICATION

(11) **EP 3 697 488 B1**
(45) Date of publication and mention of the grant of the patent: **29.05.2024**
(21) Application number: 18811913.5
(22) Date of filing: 15.10.2018
(51) Int. Cl.: A61M 25/01, A61M 25/00

(54) **CHANGING CATHETER INTO STEERING TOOL**
UMWANDLUNG EINES KATHETERS IN EIN LENKWERKZEUG
CHANGEMENT D'UN CATHÉTER EN UN OUTIL D'ORIENTATION

(30) Priority: 18.10.2017 US 201715786616
(43) Date of publication of application: 26.08.2020
(73) Proprietor: Bendit Technologies Ltd., 4951024 Petah Tikva (IL)
(72) Inventor: CABIRI, Oz, 4526611 Hod HaSharon (IL)
(74) Representative: Wright, Howard Hugh Burnby
(86) International application number: PCT/IB2018/057960
(87) International publication number: WO 2019/077461

(56) References cited:
- WO-A1-2017/158069
- US-A1- 2011 137 309
- US-A1- 2013 116 705
- US-A1- 2013 304 035
- US-A1- 2016 310 702

## Description

### FIELD OF THE INVENTION

The present invention generally relates to an assembly of a passive catheter and a distal end steering tool.

### BACKGROUND OF THE INVENTION

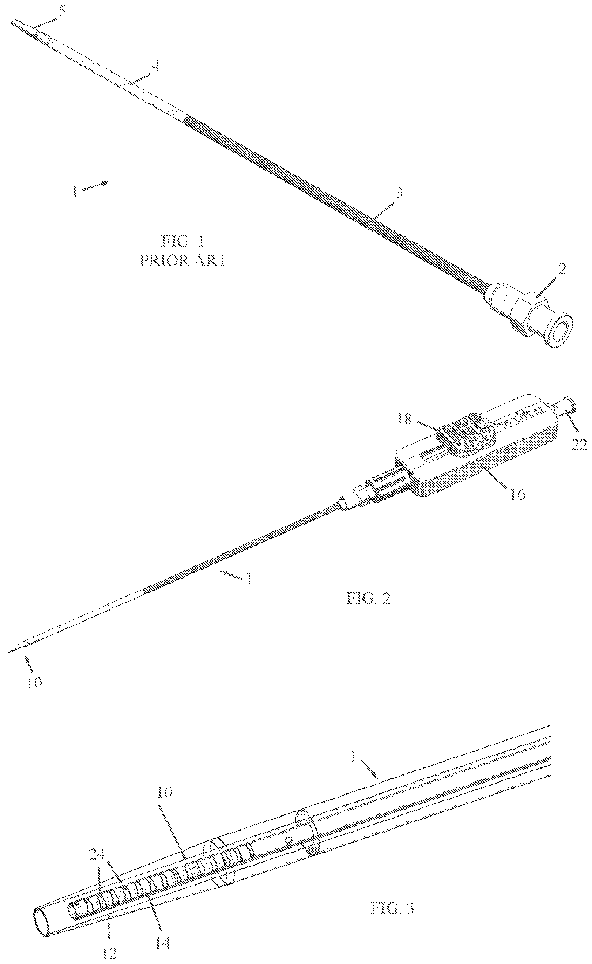

A typical prior art catheter 1 is shown in Fig. 1. Catheter 1 may have a proximal connection feature 2 (e.g., a luer lock or fluid connector). Catheter 1 includes a tube, which may have one or more segments, such as a proximal section 3, a distal section 4, and a distal tip 5, each of which has difference characteristics, such as but not limited to, dimensions, stiffness, added supportive structure, inner and outer coatings, and other features, such as a tip and radio pack.

Known catheters like catheter 1 are passive, that is, even though they may have soft distal ends, the distal end does not have the ability to bend through tortuous bends in body lumens.

US-A1-2016/310702 describes a steering tool including an internal tube disposed inside an external tube. The internal and external tubes are arranged for longitudinal axial movement relative to one another. A distal end of the internal tube is fixedly joined to a distal end of the external tube. At least one of the internal and external tubes is slotted near the distal end thereof, and the longitudinal axial movement causes bending of the distal ends of the tubes.

US-A1-2013/116705 describes a steerable medical delivery comprising: a steerable portion adapted to be steered; and an outer sheath and an inner sheath disposed within the outer sheath along a first portion of the delivery device, the outer sheath comprising a first material and the second sheath comprising a second material, wherein the first and second materials are merged together at a merged location distal to the steerable portion, forming a unitary section of material at the merged location, wherein the inner and outer tubular members are adapted to be axially moved relative to one another along the steerable portion to steer the steerable portion.

### SUMMARY OF THE INVENTION

The present invention seeks to provide an assembly of a passive catheter and a distal end steering tool, as is described more in detail hereinbelow.

In the present invention, a steering tool tube assembly is added to the catheter and changes the catheter into a steering tool catheter. The steering tool tube assembly includes inner and outer tubes whose distal ends are coupled to each other.

Some non-limiting examples of commercially available catheters which may be modified by the structure of the present invention into a steering tool catheter include:
- SOFIA Distal Access Catheter by Microvention
- CAT 6 Distal Access Catheter by Stryker
- AXS Infinity LS Long Sheath by Stryker
- FLOWGATE 2 balloon guide by Stryker
- ACE family of reperfusion catheters by Penumbra
- NEURON MAX System by Penumbra
- ARC^{™} INTRACRANIAL SUPPORT CATHETER by Medtronic
- FUBUKI guide catheter by Asahi

There is thus provided in accordance with an embodiment of the present invention an assembly comprising a catheter comprising a proximal connection feature and a tube, said tube having a proximal section, a distal section, and a tapered distal tip,a tube assembly that passes through said catheter, the tube assembly including an internal tube and an external tube whose distal ends extend to said distal section of said catheter, and said internal tube and said external tube are coupled to each other and arranged for longitudinal axial movement relative to one another, and a handle comprising a tube manipulator coupled to the tube assembly, which is operative to cause relative axial movement of the internal and external tubes and bending of a distal portion of at least one of the internal and external tubes.

In accordance with the present invention the external tube is different from the catheter. Both the internal and external tubes may pass through a distal end of the catheter or may abut against the distal end of the catheter.

In accordance with an embodiment of the present invention the tube manipulator is operative to lock the tube assembly at a bent position.

### BRIEF DESCRIPTION OF THE DRAWINGS

The present invention will be understood and appreciated more fully from the following detailed description taken in conjunction with the drawings in which:
Fig. 1 is a simplified illustration of a prior art catheter;
Fig. 2 is a simplified illustration of an assembly of a passive catheter and a distal end steering tool, in accordance with a non-limiting embodiment of the present invention, in which the added steering tool is an assembly of an internal tube and an outer tube coupled to each other and which extends from the distal end of the existing catheter;
Fig. 3 is a simplified enlarged view of one end of the assembly of Fig. 2;
Fig. 4 is a simplified illustration of an internal tube with a stopper for coupling to the distal end of the existing catheter, in accordance with another non-limiting embodiment of the present invention;
Fig. 5 is a simplified illustration of the internal tube with stopper of Fig. 4 preassembled with a passive catheter, in accordance with another non-limiting embodiment of the present invention; and
Fig. 6 is a simplified illustration of the internal tube with stopper assembled and ready to use in the system.

### DETAILED DESCRIPTION OF EMBODIMENTS

Reference is now made to Fig. 2, which illustrates an assembly of a passive catheter 1 and a distal end steering tool 10, in accordance with a non-limiting embodiment of the present invention, and to Fig. 3 which shows the structure of the steering tool 10.

As seen best in Fig. 3, steering tool 10 includes an internal tube 12 disposed inside an external tube 14. A distal end of internal tube 12 is fixedly joined to a distal end of external tube 14. The term "joined" encompasses any method for attaching the materials of the tubes together, such as but not limited to, welding, ultrasonic welding, thermal bonding, adhesive bonding, molding, and others. The internal and external tubes 12 and 14 are arranged for longitudinal axial movement relative to one another (except for their distal ends which are joined together).

Referring again to Fig. 2, steering tool 10 includes a handle 16 that has a tube manipulator 18 (also referred to as control knob 18) for causing longitudinal axial movement of one of the internal and external tubes 12 and 14 relative to one another so as to cause the distal portions of the tubes to bend or curve or otherwise deform. The same or another tube manipulator may be used to lock the tubes 12 and 14 of steering tool 10 completely or partially or not at all (i.e., unlocked so the tubes can move freely), as described in PCT Patent Application PCT/US2014/071075 or PCT/IB2017/051040. For example, control knob 18 may be a slidable knob that slides in a channel 20 and which may be lockable at different positions. In alternative embodiments, tube manipulator 18 may be a rotating element. The term "knob" encompasses any manipulative element, such us but not limited to, a knob, button, roller, switch and the like. For example, instead of being a mechanical knob, alternatively, the invention may be carried out with an electronic biasing system, in which case the control knob may be an electrical switch, such as a button or touchpad.

One or both of the internal and outer tubes may be formed with slots 24 (Fig. 3). The slots 24 may be phase-shifted with respect to one another (i.e., the slots on one tube may be phase-shifted with respect to one another and/or the slots of one tube may be phase-shifted with respect to the slots of the other tube). A phase shift manipulator (not shown, but described in PCT Patent Application PCT/IB2017/051040) may be used to apply torque to internal tube 12 or external tube 14, thereby changing the amount of phase shift between the tubes or between the slots, and thereby causing a twisted or spiral shape to the distal tips of the tubes. This provides the surgeon with limitless possibilities of shaping the distal tips to any desired, three-dimensional shape. The twisting torque also changes the stiffness of the bent portion of the tubes.

Handle 16 may include a medical device connector 22, such as a fluid connector, luer fitting and others.

As seen in Fig. 2, the added steering tool 10 passes through catheter 1 and extends to the distal end of the existing catheter 1. Catheter 1 has now been changed into a novel catheter with a distal end steering tool.

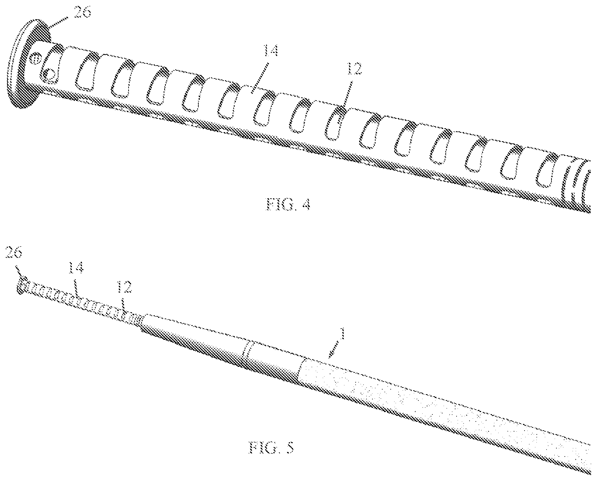

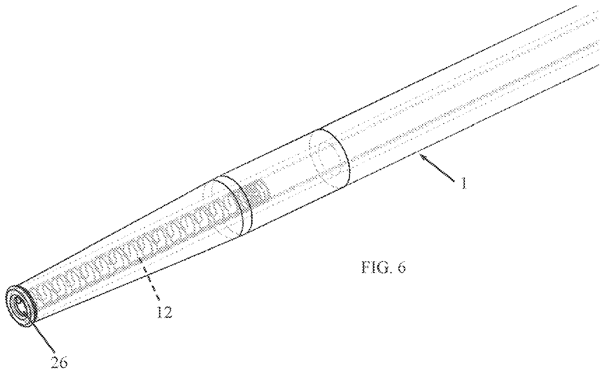

Reference is now made to Figs. 4 and 5. In this embodiment, internal tube 12 may be provided with a stopper 26 or locker or any other coupling method to the distal end of the existing catheter 1. In other words, in one embodiment, there is no external tube 14; rather the existing catheter 1 serves as the external tube and the stopper 26, which abuts against the distal end of the catheter (as seen in Fig. 6), effectively couples the distal ends of the existing catheter 1 and the internal tube 12 so that longitudinal axial movement relative to one another causes the distal ends to bend as before. In such an embodiment, there is a cost saving by not using an external tube in addition to the catheter.

Alternatively, as shown in Fig. 5, stopper 26 may also be used in the embodiment that includes external tube 14. In such an embodiment, the stopper 26 is used to couple or join the distal ends of the internal and external tubes. The assembly of the internal and external tubes may extend distally from the distal end of the catheter.

## Claims

1. An assembly comprising:
a catheter (1) comprising a proximal connection feature (2) and a tube, said tube having a proximal section (3), a distal section (4), and a tapered distal tip (5);
a tube assembly that passes through said catheter (1), said tube assembly comprising an internal tube (12) and an external tube (14) whose distal ends extend to said distal section (4) of said catheter (1);
wherein said internal tube (12) and said external tube (14) are coupled to each other and arranged for longitudinal axial movement relative to one another; and
a handle (16) comprising a tube manipulator (18) coupled to said tube assembly, which is operative to cause relative axial movement of said internal (12) and external (14) tubes and bending of a distal portion of at least one of said internal (12) and external (14) tubes.

2. The assembly according to claim 1, wherein said tube assembly passes through said catheter and said external tube (14) is different from said catheter (1), and both said internal (12) and external (14) tubes pass through a distal end of said catheter (1).

3. The assembly according to claim 1, wherein a stopper (6) is used to couple or join the distal ends of the internal and external tubes (12, 14).

4. The assembly according to claim 1, wherein said tube manipulator (18) is operative to lock said tube assembly at a bent position.

5. The assembly according to claim 2, wherein said internal (12) and external (14) tubes abut against a distal end of said catheter (1).

6. The assembly according to claim 2, wherein said internal (12) and external (14) tubes pass through a distal end of said catheter (1).

## Patentansprüche

1. Eine Anordnung, aufweisend:
einen Katheter (1), der ein proximales Verbindungsmerkmal (2) und ein Rohr aufweist, wobei das Rohr einen proximalen Abschnitt (3), einen distalen Abschnitt (4) und eine verjüngte distale Spitze (5) hat;
eine Rohranordnung, die den Katheter (1) durchläuft, wobei die Rohrandordnung ein inneres Rohr (12) und ein äußeres Rohr (14) aufweist, deren distalen Enden sich zu dem distalen Abschnitt (4) des Katheters (1) erstrecken;
wobei das innere Rohr (12) und das äußere Rohr (14) miteinander gekoppelt sind und für axiale Längsbewegung relativ zueinander angeordnet sind; und
einen Griff (16), der einen Rohrmanipulator (18) aufweist, der an die Rohranordnung gekoppelt ist und ausgelegt ist, um eine relative axiale Bewegung des inneren Rohrs (12) und des äußeren Rohrs (14) und ein Biegen eines distalen Stücks mindestens eines des inneren Rohrs (12) und des äußeren Rohrs (14) zu verursachen.

2. Die Anordnung gemäß Anspruch 1, wobei die Rohranordnung den Katheter durchläuft und das äußere Rohr (14) sich von dem Katheter (1) unterscheidet und sowohl das innere Rohr (12) als auch das äußere Rohr (14) ein distales Ende des Katheters (1) durchlaufen.

3. Die Anordnung gemäß Anspruch 1, wobei ein Stopper (6) verwendet wird, um die distalen Enden des inneren und äußeren Rohrs (12, 14) zu koppeln oder zu verbinden.

4. Die Anordnung gemäß Anspruch 1, wobei der Rohrmanipulator (18) ausgelegt ist, um die Rohranordnung an einer gebogenen Position zu verriegeln.

5. Die Anordnung gemäß Anspruch 2, wobei das innere Rohr (12) und das äußere Rohr (14) an einem distalen Ende des Katheters (1) anliegen.

6. Die Anordnung gemäß Anspruch 2, wobei das innere Rohr (12) und das äußere Rohr (14) ein distales Ende des Katheters (1) durchlaufen.

## Revendications

1. Ensemble comprenant :
un cathéter (1) comprenant une caractéristique de raccordement proximale (2) et un tube, ledit tube ayant une section proximale (3), une section distale (4) et une pointe distale progressivement rétrécie (5) ;
un ensemble de tube qui passe à travers ledit cathéter (1), ledit ensemble de tube comprenant un tube interne (12) et un tube externe (14) dont les extrémités distales s'étendent jusqu'à ladite section distale (4) dudit cathéter (1) ;
dans lequel ledit tube interne (12) et ledit tube externe (14) sont couplés entre eux et agencés pour le mouvement axial longitudinal l'un par rapport à l'autre ; et
une poignée (16) comprenant un manipulateur de tube (18) couplé audit ensemble de tube, qui est opérationnel pour provoquer le mouvement axial relatif desdits tubes interne (12) et externe (14) et la flexion d'une partie distale d'au moins l'un parmi lesdits tubes interne (12) et externe (14).

2. Ensemble selon la revendication 1, dans lequel ledit ensemble de tube passe à travers ledit cathéter et ledit tube externe (14) est différent dudit cathéter (1) et à la fois lesdits tubes interne (12) et externe (14) passent à travers une extrémité distale dudit cathéter (1) .

3. Ensemble selon la revendication 1, dans lequel une butée (6) est utilisée pour coupler ou assembler les extrémités distales des tubes interne et externe (12, 14) .

4. Ensemble selon la revendication 1, dans lequel ledit manipulateur de tube (18) est opérationnel pour verrouiller ledit ensemble de tube dans une position fléchie.

5. Ensemble selon la revendication 2, dans lequel lesdits tubes interne (12) et externe (14) viennent en butée contre une extrémité distale dudit cathéter (1).

6. Ensemble selon la revendication 2, dans lequel lesdits tubes interne (12) et externe (14) passent à travers une extrémité distale dudit cathéter (1).
